# EUROPEAN PATENT APPLICATION

(11) **EP 1 352 954 A2**
(43) Date of publication of application: **15.10.2003**
(21) Application number: 03006077.6
(22) Date of filing: 19.03.2003
(51) Int. Cl.: C12N 5/06, C07K 14/435

(54) **Method of preparing culture support factor from insects**

(30) Priority: 05.04.2002 JP 2002104118
(71) Applicant: HOKKAIDO UNIVERSITY, Sapporo 060-0808 (JP)
(72) Inventor: Hayakawa, Yoichi, Sapporo-shi,Hokkaido (JP)
(74) Representative: Hertz, Oliver, Dr.

(57) **Abstract**

A method of preparing a culture support factor for cells or tissues from an insect, comprising the steps of steam-sterilizing a worm body of a lava or pupa of the insect, and subjecting the steam-sterilized worm body to extraction with an aqueous extraction solution, thereby recovering the culture support factor in the extraction solution.

## Description

The present invention relates to a method of preparing a culture support factor useful in culturing cells and tissues of animals, in particular, insects.

A culture technology for cells and tissues plays an important role in biotechnology, more specifically, in producing useful proteins by gene recombination. Particularly, compared to bacterial cells, insect cells express a protein at a high efficiency and are expected to have a similar post-translational modification to that of mammalian cells. For these reasons, insect cells have received attention as a host for expressing various types of recombinant proteins, in other words, as a factory for producing useful proteins.

To culture such animal cells and tissues, a culture support factor needs to be added to a medium. This is a common technique in the prior art. As a culture support factor, bovine serum has been conventionally used in wide variety of fields over basic and application research. However, bovine serum varies in activity depending upon the lot, rendering the quality unstable. In addition, bovine serum is expensive. Particularly, when a useful protein is produced in a large quantity by using insect cells as a host, the expensive and unstable quality are the first problems to be overcome.

The present invention has been attained to solve the aforementioned problems and is intended to provide a method of preparing a culture support factor for culturing cells and tissues of an animal, in particular, an insect, the culture support factor being obtained with a stable quality at low cost compared to bovine serum.

The present inventors have conducted intensive studies to attain the aforementioned object. As a result, they found that a culture support factor having a high activity can be prepared from a lava or pupa of the order of *Lepidoptera* and arrived at the present invention.

To be more specific, the present invention is directed to a method of preparing a culture support factor for cells or tissues from an insect, comprising the steps of:
steam-sterilizing a worm body of a lava or pupa of the insect; and
subjecting the steam-sterilized worm body to extraction with an aqueous extraction solution, thereby recovering the culture support factor in the extraction solution.

According to a preferable aspect of the present invention, there is provided a method of preparing a culture support factor for cells or tissues from an insect, comprising the steps of:
steam-sterilizing a worm body of a lava or pupa of the insect;
crushing the steam-sterilized worm body into pieces; and
subjecting the crushed worm body pieces to extraction with an aqueous extraction solution, thereby recovering the culture support factor in the extraction solution.

This summary of the invention does not necessarily describe all necessary features so that the invention may also be a sub-combination of these described features.

The invention can be more fully understood from the following detailed description when taken in conjunction with the accompanying drawings, in which:

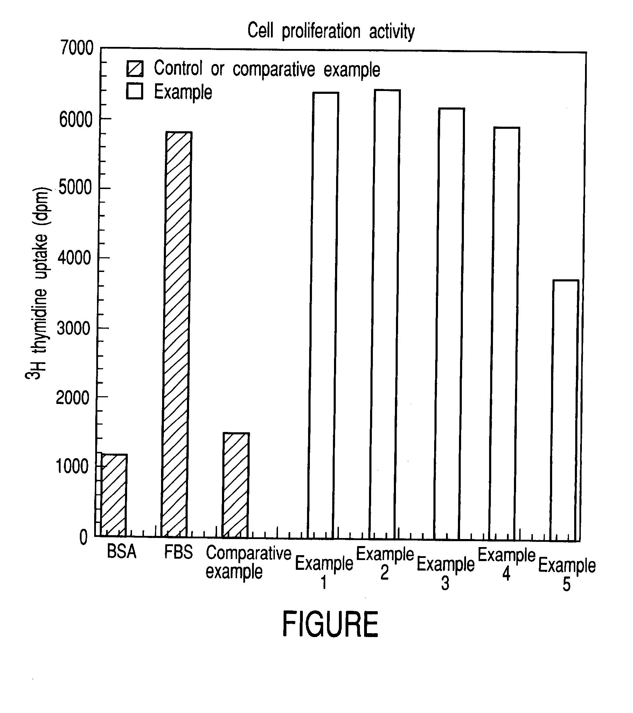

The single Figure is a graph showing the test results of a cell proliferation activity when a culture support factor prepared from the armyworm larvae by an example of the present invention is used.

The lava or pupa to be used as a raw material in the present invention is not particularly limited. Any species of insect may be used. However, a lava or pupa of an insect belonging to the order *Lepidoptera* is preferably used. Particularly preferable examples may include, but not limited to, lavae and pupae of armyworm, cutworm, and silkworm. Similar activity can be obtained when *Lucilia cuprina* is used. Therefore, insects of the order *Diptera* may be used in the present invention.

The steam sterilization step of the present invention can be performed by using a customary steam sterilization apparatus. The sterilization temperature generally ranges from 105°C to 130°C, preferably from 110°C to 130°C, and most preferably from 120°C to 125°C. The sterilization time generally ranges from 1 minute to 2 hours, more preferably from 5 minutes to 1 hour, and further preferably, from 5 minutes to 20 minutes.

The steam sterilization step plays an important role in the present invention especially when larvae ware used as raw materials. If the sterilization is performed by boiling instead of steam sterilization, the activity of the culture support factor finally recovered through extraction is rarely detected. The reason has not yet been revealed. However, it is conceivable that, in the case of steam sterilization, the culture support factor can be recovered while maintaining its activity, whereas, in the case of boiling sterilization, either a proliferation inhibitor still remains adversely effective or a proliferation support factor (culture support factor) cannot be activated.

When newly ecdyzed pupae were used as raw materials, the culture factor with a reasonable activity could be obtained by boiling. The term "newly ecdyzed" used herein means that the pupae have ecdyzed on the day of preparing the culture support factor according to the present invention, more preferably, within 4 hours from the ecdysis.

The aqueous extraction solution to be used in the present invention may be water itself. Alternatively, an aqueous solution such as a physiological saline solution may be used.

The extraction method to be used in the present invention is not particularly limited. Any extraction method known in the prior art may be used. Preferably, the worm body may be soaked in physiological saline to sterilize it, crushed into pieces, and subjected to centrifugal extraction. A means for crushing the worm body into pieces is not particularly limited. For example, a mechanical homogenization means such as a mixer may be used. Preferably, an ultrasonic crushing means may be used. As the ultrasonic crushing means, a customary ultrasonic crusher may be used.

By performing the crushing step in the presence of an aqueous extraction solution, crushing and extraction steps may be performed at the same time. Furthermore, both the steam sterilization step and the crushing step may be performed in the presence of an aqueous extraction solution. In these cases, a step of separating the residual worm body pieces from the extraction solution is preferably performed. The separation step may be performed by a known means such as filtration or centrifugal separation.

The supernatant thus obtained may be used as it is without any particular treatment. The supernatant may be added to a medium for culturing cells or tissues, in a final concentration of, for example, 3 vol% to 20 vol%, preferably, 5 vol% to 10 vol%.

The types of cells and tissues to be cultured in a medium employing the culture support factor prepared by the present invention are not particularly limited. However, preferably, insect cells or insect tissues may be favorably used. Particularly, the culture support factor is useful in culturing insect culture cells such as High 5 and Sf 9, which are used as baculovirus expression system for expressing a useful protein.

According to the present invention, an inexpensive raw material such as a lava or pupa of an insect is used and the manufacturing step is extremely simple. Therefore, a culture support factor can be obtained at low cost. By controlling operation conditions such as steam sterilization and extraction, it is possible to obtain a culture support factor of a stable quality.

### Examples

Now, the present invention will be described in more detail by way of examples.

The worm body of last-instar armyworm lavae was placed in a microtube containing physiological saline and subjected to steam sterilization in an autoclave. The temperature of the steam was set at 120°C. The sterilization was performed for 5 minutes (Example 1), 10 minutes (Example 2), 20 minutes (Example 3), one hour (Example 4), and 2 hours (Example 5). The worm body sterilized with steam in this manner was ultrasonically crushed by an ultrasonic crusher. Thereafter, centrifugal separation was performed at 20,000g for 10 minutes to obtain, as the supernatant, the solution containing the culture support factor according to the present invention.

The same manner as mentioned above was repeated except that boiling sterilization was performed for 10 minutes instead of steam sterilization. As a result, an extraction solution of a last-instar armyworm lava (comparative example) was obtained.

Culture support factors obtained in Examples 1-5 and the comparative example, as well as BSA (bovine serum albumin) and FBS (fetal bovine serum) as controls were added to Grace's insect cell culture medium in a final concentration of 5 vol% for each. Insect cells (High 5) were cultured in these mediums. The cell proliferation activity was determined by measuring the uptake of ³H thymidine added to each medium. The results are shown in Figure.

As is apparent from the results shown in Figure, it is demonstrated that the extraction solutions prepared in Examples 1 to 5 of the present invention exhibit extremely strong cell proliferation supporting activities (culture supporting activities) equivalent to that of FBS. On the other hand, the culture supporting activity of the comparative example, in which boiling sterilization is performed instead of steam sterilization, is as low as that of BSA.

It was further confirmed that the culture support factor prepared in accordance with the present invention is also useful in the passage culture of insect cells (High 5).

It should be noted that in additional experiments, newly ecdyzed armyworm pupae after 4 hours from the ecdysis were used as raw materials, and sterilization was carried out by boiling. Any other steps were conducted similarly as the examples described above. As a result, the resultant supernatant is revealed to have approximately same activity as that obtained form Examples described above.

## Claims

1. A method of preparing a culture support factor for cells or tissues from an insect, **characterized by** comprising the steps of:
steam-sterilizing a worm body of a lava or pupa of the insect; and
subjecting the steam-sterilized worm body to extraction with an aqueous extraction solution, thereby recovering the culture support factor in the extraction solution.

2. The method of preparing a culture support factor for cells or tissues from an insect, **characterized by** comprising the steps of:
steam-sterilizing a worm body of a lava or pupa of the insect;
crushing the steam sterilized worm body into pieces; and
subjecting the crushed worm body pieces to extraction with an aqueous extraction solution, thereby recovering the culture support factor in the extraction solution.

3. The method according to claim 2, **characterized in that** said step of crushing the steam-sterilized worm body into pieces is performed ultrasonically.

4. The method according to claim 2, **characterized by** further comprising the step of centrifugally separating the extraction solution from the residual crushed worm body pieces.

5. The method according to claim 1, **characterized in that** the insect belongs to the order *Lepidoptera.*

6. The method according to claim 5, **characterized in that** the insect is selected from the group consisting of armyworm, cutworm, and silkworm.

7. A method of preparing a culture support factor for cells or tissues from an insect, **characterized by** comprising the steps of:
boil-sterilizing a newly ecdyzed pupa of the insect; and
subjecting the boil-sterilized worm body to extraction with an aqueous extraction solution, thereby recovering the culture support factor in the extraction solution.

8. The method of preparing a culture support factor for cells or tissues from an insect, **characterized by** comprising the steps of:
boil-sterilizing a newly ecdyzed pupa of the insect;
crushing the boil-sterilized pupa body into pieces; and
subjecting the crushed pupa body pieces to extraction with an aqueous extraction solution, thereby recovering the culture support factor in the extraction solution.

9. The method according to claim 8, **characterized in that** said step of crushing is performed ultrasonically.

10. The method according to claim 8, **characterized by** further comprising the step of centrifugally separating the extraction solution from the residual crushed pupa body pieces.

11. The method according to claim 7, **characterized in that** the insect belongs to the order *Lepidoptera.*

12. The method according to claim 11, **characterized in that** the insect is selected from the group consisting of armyworm, cutworm, and silkworm.
